(19) **Europäisches Patentamt / European Patent Office / Office européen des brevets**

(11) **EP 4 081 246 B1**

(12) **EUROPEAN PATENT SPECIFICATION**

(45) Date of publication and mention of the grant of the patent:
**10.04.2024 Bulletin 2024/15**

(21) Application number: **20839094.8**

(22) Date of filing: **23.12.2020**

(51) International Patent Classification (IPC):
*A61K 39/00* (2006.01)  *A61P 37/08* (2006.01)
*C12N 5/078* (2010.01)

(52) Cooperative Patent Classification (CPC):
**A61K 39/0005; A61P 37/08; C12N 5/0634;**
C12N 2529/10

(86) International application number:
**PCT/EP2020/087757**

(87) International publication number:
**WO 2021/130305 (01.07.2021 Gazette 2021/26)**

(54) **COMPOSITION FOR TREATING OR PREVENTING AN ALLERGY OR ALLERGIC REACTION**

ZUSAMMENSETZUNG ZUR BEHANDLUNG ODER PRÄVENTION EINER ALLERGIE ODER EINER ALLERGISCHEN REAKTION

COMPOSITION DE TRAITEMENT OU DE PRÉVENTION D'UNE ALLERGIE OU D'UNE RÉACTION ALLERGIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **23.12.2019 EP 19219342**

(43) Date of publication of application:
**02.11.2022 Bulletin 2022/44**

(73) Proprietor: **Aposcience AG**
**1200 Wien (AT)**

(72) Inventors:
• **ANKERSMIT, Henrik Jan**
**1060 Wien (AT)**
• **MILDNER, Michael**
**3040 Neulengbach (AT)**

(74) Representative: **Pföstl, Andreas**
**Schwarz & Partner Patentanwälte**
**Wipplingerstraße 30**
**1010 Wien (AT)**

(56) References cited:
**WO-A1-2019/121989**

• **LUCIAN BEER ET AL: "Analysis of the Secretome of Apoptotic Peripheral Blood Mononuclear Cells: Impact of Released Proteins and Exosomes for Tissue Regeneration", SCIENTIFIC REPORTS, vol. 5, no. 1, 16 November 2015 (2015-11-16), XP055589420, DOI: 10.1038/srep16662**
• **MARTINE HEYMAN ET AL: "Mononuclear cells from infants allergic to cow's milk secrete tumor necrosis factor [alpha], altering intestinal function", GASTROENTEROLOGY, vol. 106, no. 6, 1 June 1994 (1994-06-01), pages 1514-1523, XP055701022, US ISSN: 0016-5085, DOI: 10.1016/0016-5085(94)90405-7**
• **LEE NA RAE ET AL: "Cytokine secreted by S100A9 via TLR4 in monocytes delays neutrophil apoptosis by inhibition of caspase 9/3 pathway", CYTOKINE, ACADEMIC PRESS LTD, PHILADELPHIA, PA, US, vol. 86, 25 July 2016 (2016-07-25), pages 53-63, XP029682223, ISSN: 1043-4666, DOI: 10.1016/J.CYTO.2016.07.005**
• **HAK SUN YU ET AL: "Culture supernatant of adipose stem cells can ameliorate allergic airway inflammation via recruitment of CD4+CD25+Foxp3 T cells", STEM CELL RESEARCH & THERAPY, vol. 8, no. 1, 23 January 2017 (2017-01-23), XP055701050, DOI: 10.1186/s13287-016-0462-5**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- Monica Baiula ET AL: "Mesenchymal stem cell secretome to control inflammation in allergic conjunctivitis", Eye Sci, 1 January 2015 (2015-01-01), pages 140-142, XP055701183, DOI: 10.3978/j.issn.1000-4432.2015.11.18 Retrieved from the Internet: URL:https://ykxb.amegroups.com/article/view/3531/4229 [retrieved on 2020-06-04]
- CARL HARRELL ET AL: "Molecular Mechanisms Responsible for Therapeutic Potential of Mesenchymal Stem Cell-Derived Secretome", CELLS, vol. 8, no. 5, 16 May 2019 (2019-05-16), page 467, XP055701139, DOI: 10.3390/cells8050467
- THOMAS HAIDER ET AL: "The secretome of apoptotic human peripheral blood mononuclear cells attenuates secondary damage following spinal cord injury in rats", EXPERIMENTAL NEUROLOGY, vol. 267, 1 May 2015 (2015-05-01), pages 230-242, XP055700864, AMSTERDAM, NL ISSN: 0014-4886, DOI: 10.1016/j.expneurol.2015.03.013
- TANJA WAGNER ET AL: "Different pro-angiogenic potential of [gamma]-irradiated PBMC-derived secretome and its subfractions", SCIENTIFIC REPORTS, vol. 8, no. 1, 1 December 2018 (2018-12-01), XP055589646, DOI: 10.1038/s41598-018-36928-6
- LICHTENAUER MICHAEL ET AL: "Secretome of apoptotic peripheral blood cells (APOSEC) confers cytoprotection to cardiomyocytes and inhibits tissue remodelling after acute myocardial infarction: a preclinical study", BASIC RESEARCH IN CARDIOLOGY, STEINKOPFF-VERLAG, DA, vol. 106, no. 6, 1 November 2011 (2011-11-01), pages 1283-1297, XP002777988, ISSN: 1435-1803
- BEER LUCIAN ET AL: "Peripheral blood mononuclear cell secretome for tissue repair", APOPTOSIS, LONDON, GB, vol. 21, no. 12, 1 October 2016 (2016-10-01), pages 1336-1353, XP036085112, ISSN: 1360-8185, DOI: 10.1007/S10495-016-1292-8 [retrieved on 2016-10-01]
- MARIA LAGGNER ET AL: "Therapeutic potential of lipids obtained from [gamma]-irradiated PBMCs in dendritic cell-mediated skin inflammation", EBIOMEDICINE, vol. 55, 1 May 2020 (2020-05-01), page 102774, XP055703846, ISSN: 2352-3964, DOI: 10.1016/j.ebiom.2020.102774

**Description**

TECHNICAL FIELD

The present invention relates to compositions and methods for treating and preventing disorders caused by the uptake of allergens to a human or mammal body.

BACKGROUND ART

During the sensitization phase of allergies, professional antigen-presenting cells phagocytose antigens (with regard to allergies referred to as allergens) and cross-present processed antigens to $T_H2$ lymphocytes. These cells respond to allergen-encounter by release of interleukin 4 and interact with B lymphocytes. Activated B cells, together with IL-4, produce type E immunoglobulins (IgE) directed against the allergen. Secreted IgE is bound by IgE-specific receptors present on, e.g., mast cells. These immune cells are thereby sensitized to the allergen. Re-exposure results in binding of the allergen to IgE-coated immune cells, which, after receptor crosslinking, causes activation of sensitized cells. Activated mast cells respond by vast release of immune mediators, such as histamine, cytokines, and pro-inflammatory lipid species, from intracellular granules. Released mediators have pleiotropic effects, including vasodilation, mucus secretion, and nerve stimulation, which result in itchiness and redness. These symptoms might be local or systemic.

Mast cells predominantly reside in tissues repeatedly confronted with pathogenic stimuli, such as the skin, the airways, and the gastrointestinal tract. Commonly, mast cells are activated by immunologic signals, such as Fcγ receptors crosslinked by antigen/IgE complexes, proteins of the complement system, or toll-like receptor agonists. Mast cells respond to activation with the vast release of immune mediators. Mast cells are best known for their role in IgE-dependent allergic reactions and targeting mast cell function, such as degranulation, might help alleviating most painful symptoms associated with allergic reactions.

HAK SUN YU ET AL: "Culture supernatant of adipose stem cells can ameliorate allergic airway inflammation via recruitment of CD4+CD25+Foxp3 T cells", STEM CELL RESEARCH & THERAPY, vol. 8, no. 1, 23 January 2017 discloses that the supernatant from cultured adipose stem cells can ameliorate allergic airway inflammation.

Baiula ET AL: "Mesenchymal stem cell secretome to control inflammation in allergic conjunctivitis", Eye Sci, 1 January 2015, pages 140-142teaches that the secretome of mesenchymal stem cells is effective to control inflammation in allergic conjunctivitis.

It is an object of the present invention to provide methods and means to prevent or treat allergic reactions caused by allergens administered to a human or mammal body.

SUMMARY OF THE INVENTION

The present invention relates to a composition comprising a supernatant of a peripheral blood mononuclear cell (PBMC) cell culture for use in the treatment or prevention of an allergy or an allergic reaction caused by the administration of at least one food and/or inhalation allergen or by the systemic administration of at least one drug to a human or mammal body, wherein the PBMCs are subjected to ionizing radiation before or during cultivation.

It turned surprisingly out that a supernatant of a PBMC culture can be used to treat or prevent allergies and allergic reactions caused by food or inhalation allergens which are administered to a human or mammal. Furthermore, the composition of the present invention can also be used to treat or prevent allergies or allergic reactions caused by the systemic administration of at least one allergen.

For a long time, stem cell-based therapies have been considered a promising tool for regeneration of various injured tissues and organs. Ever since, numerous works have challenged this concept by reporting that secreted factors, rather than the cells themselves, exerted the observed regenerative effect. Peripheral blood mononuclear cells (PBMCs) represent, in contrast to stem cells, an attractive and easily accessible source for cellular secretomes with versatile features. Though immunomodulatory effects have been attributed to the secretome of preferably γ-irradiated PBMC (Aposec), it is surprising that supernatants of PBMC cultures show beneficial effects with regard to allergic reactions and allergy, in particular when the triggering allergens.

A further aspect of the present invention relates to a method for treating or preventing an allergy or an allergic reaction caused by an uptake of at least one allergen to a human or mammal body comprising the step of administering a composition as defined herein.

Another aspect of the present invention relates to a method for determining the suitability of a composition as defined herein to be used in the treatment or prevention of a an allergy or allergic reactions caused the administration of at least one food and/or inhalation allergen or by the systemic administration of at least one drug to a human or mammal body comprising the steps of

a) comparing skin regions of a mammal contacted with an allergen with skin regions of the same mammal contacted with the allergen and the composition as defined in any one of claims 1-7 or 9,
b) identifying a difference between said regions and
c) determining whether the composition is suited to treat or prevent said disorder or disease.

Any references in the description to methods of treatment or diagnostic refer to the compounds, pharmaceutical compositions and medicaments of the present invention for use in a method for treatment of the human (or animal) body by therapy or for diagnostic.

BRIEF DESCRIPTION OF THE FIGURES

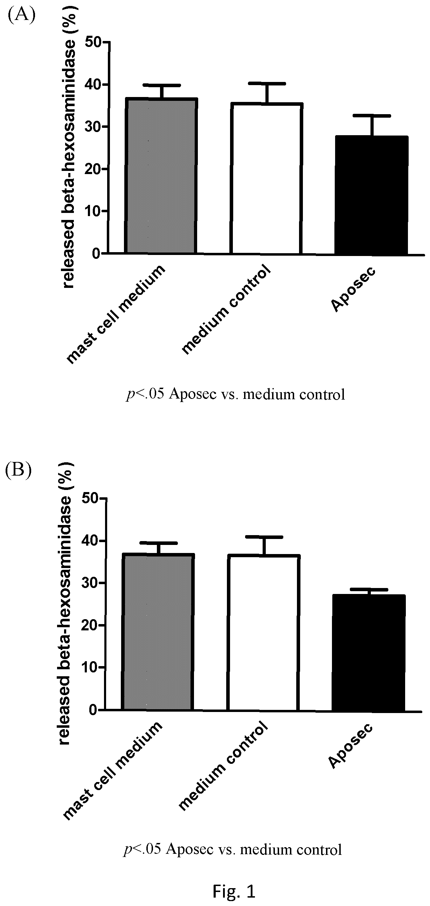

Fig. 1 shows that MNCaposec prevents compound 48/80- and IgE/anti-IgE-induced mediator release by primary human mast cells. Mast cell degranulation assessed by released beta-hexaminidase upon (A) compound 48/80 and (B) IgE/anti-IgE stimulation of primary human mast cells. Asterisks indicate $p<.05$ of Aposec compared to medium control (CellGro). Mast cell medium refers to medium routinely used to culture mast cells (DMEM alone).

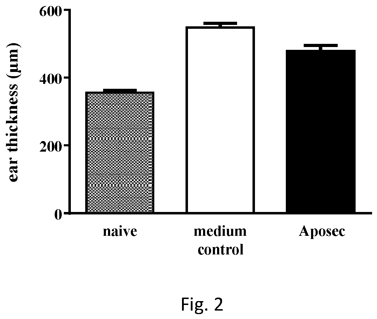

Fig. 2 shows that PBMC secretome Aposec alleviates ear swelling in DNFB-induced hypersensitivity. Ear thickness assessed by micrometer-assisted measurements 24 hours post DNFB re-challenge. $p<0.05$ Aposec vs. medium control.

DESCRIPTION OF EMBODIMENTS

The present invention relates to a composition comprising a supernatant of a PBMC cell culture for use in the treatment or prevention of an allergy or an allergic reaction caused by the administration of at least one food and/or inhalation allergen or by the systemic administration of at least one drug to a human or mammal body.
Cells, in particular mammalian cells, are known to secrete numerous substances during cultivation into a cell culture medium. The so obtained conditioned culture media can be used in the treatment and/or prevention of various diseases and disorders. For instance, WO 2010/070105 and WO 2010/079086 disclose conditioned culture media ("supernatants") which are obtained by cultivation of PBMCs and which can be used in the treatment of various inflammatory conditions. Hence, "a supernatant of a peripheral blood mononuclear cell (PBMC) cell culture", as used herein, refers to any supernatant obtainable by cultivating PBMCs *in vitro* in a culture medium. After the cultivation step the cultivated PBMCs are removed from the culture medium in order to obtain the substantially cell-free, preferably completely cell-free, supernatant. The supernatant of the PBMC culture comprises next to components of the cultivation medium substances produced and secreted by the PBMCs and/or even lysed PBMCs. "Supernatant" can be used interchangeable with conditioned culture medium obtainable by cultivating PBMCs.
The supernatant of the present invention is obtainable by cultivating PBMCs, which are subjected to ionizing radiation before or during cultivation. The ionizing radiation is preferably gamma radiation.
"An allergy or an allergic reaction caused by the administration of at least one food and/or inhalation allergen or by the systemic administration of at least one drug to a human or mammal body", as used herein, refers to any adverse reaction caused by the administration of a food and/or inhalation allergen into a human or mammal body. Food allergens are usually administered orally to a human or mammal body. Inhalative allergens are typically administered by inhalation through the nose or mouth into the lung and the airways. Some drugs used to treat human and mammal bodies can cause allergic reactions once administered systemically to such bodies. Such drugs can be administered orally, by inhalation, parenterally or any other route of administration to human or mammal bodies resulting in a systemic distribution of the drug within such bodies. Parenteral administration of a drug may include intramuscular, intraperitoneal, intravenous and other routes of administration. Topical administration of a drug allergen or any other allergen on the skin does not result in a systemic spread and hence systemic administration to a human or mammal body.
Allergens causing allergy or allergic reactions when administered to a human or mammal body include food allergens, drug allergens, inhalation allergens (e.g. pollen, chemical substances) and all kind of allergens which are administered/introduced into a human or mammal body. Hence, typical allergic reactions caused by the administration of allergens to a human or mammal body include, among others, runny nose, trouble breathing, nausea, diarrhea, nosebleeds, ear problems, wheezing, coughing or even anaphylaxis, when the allergen is introduced, for instance, into the blood stream.
"Allergen", as used herein, refers to an antigen that is capable of stimulating a hypersensitivity reaction in a human and/or mammal body through immunoglobulin E (IgE) responses typically resulting in an excessive release of histamine from mast cells. The term "allergen" includes - if the allergen is of biological origin and is a protein or polypeptide - also fragments of such naturally occurring allergens when these fragments exhibit similar effects as the naturally occurring allergen in regard to the release of histamine within a human or mammal body.

The terms "preventing" and "prevention", as used herein, refer to the prevention or inhibition of the recurrence, onset and development of an allergy or a symptom thereof in a human and mammal body resulting from the administration of the supernatant according to the present invention. In some embodiments "preventing" and "prevention" refers to the reduction of the risk to develop an allergy against specific allergens. The term "preventing" covers measures not only to prevent the occurrence of an allergy, but also to arrest its progress and reduce its consequences once established.

The terms "treatment" and "treating", as used herein, refer to the reduction or inhibition of the progression and duration of an allergy, the reduction or amelioration of the severity of the allergy and the amelioration of one or more symptoms thereof. "Treatment" encompasses also the improvement and/or reversal of the symptoms of an allergy or allergic reactions. The term "treatment" refers to both therapeutic treatment and prophylactic measures. For example, those who may benefit from treatment with compositions and methods of the present invention include those already with an allergy as well as those in which the allergy is to be prevented.

According to another preferred embodiment of the present invention the at least one allergen is a biological or chemical allergen.

Biological allergens include allergens derived from a biological system like plants, animals (e.g. insects, arachnids) or microbes (e.g. moulds, bacteria). Such allergens are in most cases proteins, polypeptides or peptides. Biological allergens are well known in the art and disclosed in various databases like **http://www.allergen.org/** or **http://www.allergome.org/.**

According to a further preferred embodiment of the present invention the biological allergen is selected from the group consisting of an animal allergen, a plant allergen, a mold allergen, or a bacterial allergen.

Animal allergens are allergens that include one or more compounds found in animals, including both vertebrates and invertebrates. Vertebrate animal allergens that may be present in mixed allergen compositions include avian allergens, such as egg allergens, e.g., nGal d 1 Ovomucoid, n Gal d 2 Ovalbumin, nGal d 3 Conalbumin, egg white complete allergen, etc.; mammalian allergens, such as milk allergens, e.g., nBos d 4 alpha-lactalbumin, nBos d 5 beta-lactoglobulin, nBos d 8 Casein, nBos d Lactoferrin, milk complete allergen, etc.; fish allergens, e.g., rCyp c 1, rGad c 1, cod complete allergen, white fish allergens, pink fish allergens, etc. Invertebrate animal allergens that may be present in mixed allergen compositions include: crustacean allergens, such as shrimp allergens, e.g., rPen a 1 tropomyosin, shrimp complete allergen, etc.; insect allergens, e.g., bee sting venom allergen, wasp sting venom allergen, mosquito bite allergen, etc.; and the like. Inhalative animal allergens may include cats or dog fur and dander, cockroach calyx and dust mite excretion.

Plant allergens are allergens that include one or more compounds found in plants. Plant allergens of interest include: wheat allergens, e.g., rTri a 19 Omega-5 Gliadin, wheat complete allergen, gliadin wheat, rTri a 14 LTP, etc.; kiwi allergens, e.g., rAct d 8 PR-10, kiwi complete allergen, etc.; celery allergens, e.g., rApi g 1.01 PR-10, rPhl p 12, celery complete allergen, CCD MUXF3 from Bromelain, etc.; soy allergens, e.g., rGly m 4 10 PR-10, soy complete allergen, nGly m 5 Beta-conglycinin, nGly m 6 Glycinin, etc.; stone fruit allergens, e.g., f419, f420, f421, f95, f242, o214 rPru p 1 PR-10, rPru p 3 LTP, stone fruit primary complete allergen, CCD MUXF3 from Bromelain, etc.; oat allergens, e.g., oat component allergens, oat complete allergen, etc.; sesame allergens, e.g., sesame seed component allergens, sesame see complete allergen, etc. Plant allergens include also inhalative allergens like pollen allergens. Such allergens may include birch pollen allergens (e.g. Bet v 1), grass pollen allergens (e.g. Phl p 1), ryegrass and timothy-grass allergens.

Allergens include allergens isolated from natural sources or recombinantly or chemically produced allergens.

According to another preferred embodiment of the present invention the PBMC cell culture comprises monocytes, T cells, B cells and/or NK cells.

According to a further preferred embodiment of the present invention the PBMCs cells are cultivated in a cell culture medium selected from the group consisting of a cell growth medium, preferably CellGro medium, more preferably Cellgro GMP DC medium, RPMI, DMEM, X-vivo and Ultraculture.

The PBMCs of the PBMC cell culture are subjected to ionizing radiation before or during cultivation. In addition to these stress inducing conditions the PBMCs can be subjected to further stress. Hence, the PBMCs may be subjected to one or more further stress inducing conditions before or during cultivation.

The term "under stress inducing conditions", as used herein, refers to cultivation conditions leading to stressed cells. Conditions causing stress to cells include among others heat, chemicals, radiation, hypoxia, osmotic pressure etc.

Additional stress to the cells of the present invention leads to a further increase of the expression and secretion of substances beneficial for treating inflammatory skin conditions, in particular skin conditions associated with ischemia.

Further stress inducing conditions may include hypoxia, ozone, heat (e.g. more than 2°C, preferably more than 5°C, more preferably more than 10°C, higher than the optimal cultivation temperature of PBMCs, i.e. 37°C), radiation (e.g. UV radiation, gamma radiation), chemicals, osmotic pressure (i.e. osmotic conditions which are elevated at least 10% in comparison to osmotic conditions regularly occurring in a body fluid, in particular in blood) or combinations thereof.

Hence, a further stress inducing condition can be selected from the group consisting of UV radiation, hypoxia, ozone, heat, osmotic pressure and pH shift.

According to another preferred embodiment of the present invention the PMCs are subjected to an ionizing radiation, preferably gamma radiation, at a dose of at least 10 Gy, preferably at least 20 Gy, more preferably at least 40 Gy, more preferably at least 50 Gy.

According to a preferred embodiment of the present invention the PBMCs are cultivated for at least 4 h, preferably for at least 6 h, more preferably for at least 12 h, before isolating its supernatant.

According to another preferred embodiment of the present invention the composition of the present invention is administered before, during and/or after the onset of allergic reactions and/or exposure to at least one allergen.

The composition of the present invention can be administered at different stages of the allergic reaction or even before the reaction occurs. In a particular preferred embodiment of the present invention the composition may administered before the human or mammal body is exposed to an allergen. It turned surprisingly out that the composition of the present invention is able to prevent the uptake of allergens or fragments thereof to antigen presenting cells. Hence, if such an uptake can be prevented, the allergen or fragments thereof will not be presented to the immune system of a human or mammal.

According to a preferred embodiment of the present invention the PCBMC cell culture comprises $1\times10^5$ to $1\times10^8$ PBMCs/ml, preferably $1\times10^6$ to $1\times10^7$ PBMCs/ml, more preferably $2\times10^6$ to $5\times10^6$ PBMCs/ml.

It turned out that the composition of the supernatant obtainable by cultivating PBMCs shows advantageous properties if a certain amount of PBMCs per ml cell culture medium is cultivated.

According to another preferred embodiment of the present invention 0.1 to 5 ml supernatant/kg body weight, preferably 0.3 to 3 ml/kg body weight, more preferably 0.5 to 2 ml/kg body weight, more preferably 0.8 to 1.2 ml/kg body weight, is administered to a human or mammal body.

The composition of the present invention comprises supernatant in an amount sufficient to treat or prevent allergies and allergic reactions. The volume of supernatant administered per kg body weight to a human or mammal body as indicated above refers directly to the supernatant. If the volume is too large to be administered to a human or mammal body, this volume can be reduced by, e.g., lyophilisation. Hence, the volume of the composition of the present invention to be administered may be lower than that indicated for the supernatant. A person skilled in the art knows which volumes can be administered using a specific administration route.

According to a preferred embodiment of the present invention the composition is administered by inhalation, topically, orally, sublingually, buccally, subcutaneously or intravenously.

The composition of the present invention may comprise pharmaceutically acceptable excipients such as diluents, stabilizers, carriers etc. Depending on the dosage form the preparation according to the present invention comprises the respective ingredients. Methods for preparing the same are well known to the skilled artisan.

In order to increase the shelf-life of the composition according to the present invention the supernatant or even the complete composition may be lyophilised. Methods for lyophilising such preparations are well known to the person skilled in the art.

Prior its use the lyophilised preparation can be contacted with water or an aqueous solution comprising buffers, stabilizers, salts etc.

According to another preferred embodiment of the present invention the mammal is a horse, a dog, a cat, or a camel.

The composition of the present invention can be used to treat any kind of mammal. However, the aforementioned mammal are most preferred.

Another aspect of the present invention relates to a method for treating or preventing an allergy or an allergic reaction caused by an uptake of at least one allergen to a human or mammal body comprising the step of administering a composition as defined above.

A further aspect of the present invention relates to a method for determining the suitability of a composition as defined above to be used in the treatment or prevention of an allergy or allergic reactions caused by an uptake of at least one allergen to a human or mammal body comprising the steps of

a) contacting at least two skin regions on a mammal with an allergen,
b) administering a composition as defined above to at least one of said skin regions, wherein at least one of said skin regions is not treated with said composition,
c) comparing the skin regions contacted with the allergen with the skin regions contacted with the allergen and said composition,
d) identifying a difference between said regions and
e) determining whether the composition is suited to treat or prevent said disorder or disease.

In order to test whether the composition of the present invention can be used for treating or preventing an allergy or an allergic reaction caused by the administration of at least one food and/or inhalation allergen or by the systemic administration of at least one drug to a human or mammal body, a scratch assay may be used. If the composition of the present invention is able to reduce the allergic reactions of the scratch site compared to an untreated scratch site or a scratch site treated with a negative control composition, the composition of the present invention can be administered to a human or mammal body in need thereof.

EXAMPLES

## Example 1: Impaired mast cell degranulation by Aposec

*Materials and methods*

Aposec manufacturing

Secretomes of PBMCs ("Aposec") were produced as described in Wagner T et al. (Sci Rep. 2018;8(1):18016). Briefly, PBMCs were obtained by Ficoll-Paque PLUS (GE Healthcare, USA)-assisted density gradient centrifugation and adjusted to a concentration of $2.5 \times 10^7$ cells/mL. Subsequently, cells were exposed to 60 Gy Caesium 137 $\gamma$-irradiation (IBL 437C, Isotopen Diagnostik CIS GmbH, Germany), and cultured in phenol red-free CellGenix GMP DC medium (CellGenix GmbH, Germany) for 24 $\pm$ 2 hours. Cells and cellular debris were removed by centrifugation and supernatant was passed through a 0.2 $\mu$m filter. Viral clearing was performed using Theraflex methylene blue technology (MacoPharma, France) and by $\gamma$-irradiation of the lyophilized powder (25,000 Gy, Gammatro 1500, Mediscan, Austria) as described previously (Haider T et al. Exp Neurol. 2015;267:230-42). Sterile lyophilisate was routinely cryopreserved at -80 °C.

Isolation and *in vitro* maintenance of primary human mast cells

Skin and subcutaneous fat tissues used for mast cell isolation were obtained from patients undergoing abdominoplasty. Subcutaneous tissue and reticular dermis were removed and remaining tissues were cut into small pieces and subjected to enzymatic digestion (2.4 U/mL dispase II from *Bacillus polymyxa,* Roche, Switzerland) at 4 °C overnight. After removal of the epidermis, dermal tissues were digested in collagenase I (Gibco, Thermo Fisher Scientific, USA) at 37 °C for 2 hours. $CD117^+$ mast cells were enriched by magnetic cell sorting technology (MACS System, Miltenyi Biotec, Germany) as suggested by the manufacturer. To increase purity of isolated cells, the isolation procedure was repeated one more time using $CD117^+$ cells from the first isolation run. CD117+ mast cells were cultured in DMEM supplemented with 10 % (vol/vol) heat-inactivated fetal calf serum (both Gibco), 1 % (vol/vol) penicillin/streptomycin (Biochrom, Germany), and 100 ng/mL recombinant human stem cell factor (PeproTech, USA).

Compound 48/80-induced degranulation of primary human mast cells

Primary human mast cells were seeded at a density of 50,000 to 100,000 per well in flat-bottom 96 well plates in 50 $\mu$L DMEM without colored pH indicator supplemented with serum and SCF as described above. Cells were pretreated with 50 $\mu$L Aposec or 50 $\mu$L medium control (CellGenix) overnight. On the following day, cells were carefully washed with 100 $\mu$L HEPES (N-2-hydroxyethylpiperazine-N-2-ethane sulfonic acid; Thermo Fisher Scientific) and degranulation was induced by addition of 100 $\mu$L HEPES containing 50 pg/mL compound 48/80 (Sigma Aldrich, USA). For non-stimulated controls, HEPES was added. Cells were incubated for 1 hour at 37 °C with normal ambient $CO_2$. Fifty $\mu$L supernatant were separated and preserved and cells were lysed in 100 $\mu$L 0.1 % triton X-100 (Sigma Aldrich).

IgE/anti-IgE-induced degranulation of primary human mast cells

Primary human mast cells were seeded at a density of 50,000 to 100,000 per well in flat-bottom 96 well plates in 50 $\mu$L DMEM without colored pH indicator supplemented with serum and SCF as described above. Cells were pretreated with 50 $\mu$L Aposec, 50 $\mu$L medium control (CellGenix), or 50 $\mu$L DMEM (mast cell medium) and additionally stimulated with 100 ng/mL human IgE (myeloma, Merck KGaA, Germany) overnight. On the following day, cells were carefully washed with 100 $\mu$L HEPES (N-2-hydroxyethylpiperazine-N-2-ethane sulfonic acid; Thermo Fisher Scientific) and degranulation was induced by addition of 100 $\mu$L HEPES containing 5 $\mu$g/mL anti-IgE antibody (Jackson ImmunoResearch Laboratories, Inc., USA). For non-stimulated controls, HEPES was added. Cells were incubated for 1 hour at 37 °C with normal ambient $CO_2$. Fifty $\mu$L supernatant were separated and preserved and cells were lysed in 100 $\mu$L 0.1 % triton X-100 (Sigma Aldrich).

Reagents for beta-hexosaminidase assay

The substrate solution for the beta-hexosiminidase assay was prepared by dissolving 8.9 g disodium-hydro-

genphosphate dihydrate ($Na_2HPO_4 \cdot 2H_2O$) and 650 mg p-nitro-N-acetyl-beta-D-glucosamide in 400 mL double distilled water and adjusting the pH to 4.5 with 0.4 M citric acid (all reagents Sigma Aldrich). The stop solution consists of 15.02 g glycine in 900 mL aqua bidest with a pH adjusted to 10.7 using 3 M sodium hydroxide.

Beta-hexosaminidase assay

Fifty $\mu$L substrate solution were added to 50 $\mu$L supernatants and 50 $\mu$L triton-lysed cells, respectively, and samples were incubated at 37 °C with ambient $CO_2$ for 90 minutes. Seventy-five $\mu$L stop buffer were added and optical density at 405 nm was determined by the plate-reading luminometer LUMIstar OPTIMA Reader (BMG LABTECH, Ortenberg, Germany) using FlUOstar OPTIMA software (version 1.20-0, BMG LABTECH).

Data and statistical analyses

The percentage of released beta-hexosaminidase is calculated using the following formula:

$$\% = \frac{2 \times \text{enzyme in supernatant}}{1/2 \times \text{ enzyme in supernatant} + 4 \times \text{enzyme in cell lysate}} \times 100$$

Data are presented as arithmetic means of technical replicates and standard error of the mean. Statistical analysis was performed by one-tailed t test comparing the two groups Aposec versus medium control with $p<.05$ considered statistically significant.

*Results*

Aposec abrogates forced mast cell degranulation

In order to determine whether Aposec is capable of preventing mast cell degranulation, mast cells were pretreated with Aposec or medium control and beta-hexosaminidase release of primary human mast cells after stimulation with compound 48/80 and IgE/anti-IgE was assessed. Aposec remarkably prevented both compound 48/80- as well as IgE/anti-IgE-induced mediator release compared to medium control (27.9 + 3.6% and 27.3 + 1.1% released $\beta$-hexaminidase with Aposec versus 35.7 $\pm$ 3.4% and 36.7 + 3.1% released mediator with control medium, after compound 48/80 and IgE/anti-IgE stimulus, respectively, both $p<.05$ aposec vs. control) (Figure 1). By comparison, the control medium displayed no effect on mast cell degranulation compared to mast cell medium [36.7 $\pm$ 2.3 and 36.9 $\pm$ 1.9 released mediator with mast cell medium after compound 48/80 and IgE/anti-IgE stimulation, respectively. $p>.05$ mast cell medium versus medium control in (A) and (B)].

*Conclusion*

The present data demonstrate that Aposec effectively prevents mediator release by primary human mast cells when stimulated with compound 48/80 and IgE/anti-IgE. While enzyme release was reduced by more than 20 % after chemical stimulation, beta-hexosaminidase release after IgE/anti-IgE treatment was diminished by more than 25 % with Aposec compared to medium control. Taken together, these data suggest the use of Aposec for treating mast cell degranulation-mediated allergic reactions.

**Example 2: Alleviated symptoms of allergic hypersensitivity by application of Aposec *in vivo***

*Background*

Over the past decades, extensive research on allergic hypersensitivity pathology has contributed to a better understanding of the immunologic reactions. Yet, the complex and multifaceted disease etiology represents a major obstacle for development of effective novel therapeutic interventions and clinical treatment options remain limited to date. As potent, anti-inflammatory effects of Aposec have been described previously, the potential of Aposec to relieve allergic hypersensitivity related symptoms was examined.

*Materials and methods*

Mouse model

1-Fluoro-2,4-dinitrobenzene (DNFB, Sigma-Aldrich) served as an allergen to induce inflammatory conditions in C57BL/6 mice. Twenty µL 0.25% (vol/vol) DNFB in olive oil were administered on days 0 and 1. Ears were daily treated with Aposec, while contralateral ears received vehicle medium for 6 consecutive days starting from day 0.

Micrometric measurements

Twenty-four hours post DNFB re-challenge, ear thickness was assessed using electronic digital micrometer (0-25 mm, Marathon Management Inc, USA), measuring thickness of the outer two-thirds of the ear. Measurements were performed in quadruplicates.

Statistical analysis

Data were statistically evaluated using GraphPad Prism 6 software (GraphPad Software Inc.). One-tailed, paired t test was performed to compare Aposec versus control medium, p-values below 0.05 were considered statistically significant. Data are presented as arithmetic means of biological replicates and standard error of the mean.

*Results*

Aposec mitigates allergen-induced tissue swelling

Murine DNFB-induced hypersensitivity was used as a model to study anti-inflammatory effects of Aposec in allergic reactions *in vivo.* The degree of ear swelling, reflecting severity of immune response, was found remarkably reduced by Aposec compared to medium control 24 hours post DNFB re-exposure ($553.9 \pm 12.7$ µm thickness of vehicle-treated ears versus $472.4 \pm 47.3$ µm with Aposec, $p<.05$ Aposec versus medium control. naïve ears: $355 \pm 12.7$ µm thickness) (Figure 2).

*Conclusion*

These data show that application of Aposec effectively prevented tissue swelling following allergen re-exposure. These findings indicate that Aposec represents a promising candidate for treating symptoms related to allergic reactions.

**Claims**

1. Composition comprising a supernatant of a peripheral blood mononuclear cell (PBMC) cell culture for use in the treatment or prevention of an allergy or an allergic reaction caused by the administration of at least one food and/or inhalation allergen or by the systemic administration of at least one drug to a human or mammal body, wherein the PBMCs are subjected to ionizing radiation before or during cultivation.

2. Composition for the use according to claim 1, wherein the at least one allergen is a biological or chemical allergen.

3. Composition for the use according to claim 2, wherein the biological allergen is selected from the group consisting of an animal allergen, a plant allergen, a mold allergen, or a bacterial allergen.

4. Composition for the use according to any one of claims 1 to 3, wherein the PBMC cell culture comprises monocytes, T cells, B cells and/or NK cells.

5. Composition for the use according to any one of claims 1 to 4, wherein the PBMCs are cultivated in a cell culture medium selected from a cell growth medium.

6. Composition for the use according to any one of claims 1 to 5, wherein the PMCs are subjected to an ionizing radiation at a dose of at least 10 Gy, preferably at least 20 Gy, more preferably at least 40 Gy, more preferably at least 50 Gy.

**7.** Composition for the use according to any one of claims 1 to 6, wherein the PBMCs are cultivated for at least 4 h, preferably for at least 6 h, more preferably for at least 12 h, before isolating its supernatant.

**8.** Composition for the use according to any one of claims 1 to 7, wherein the composition is administered before, during and/or after the onset of allergic reactions and/or exposure to at least one allergen.

**9.** Composition for the use according to any one of claims 1 to 8, wherein the PCBMC cell culture comprises $1 \times 10^5$ to $1 \times 10^8$ PBMCs/ml, preferably $1 \times 10^6$ to $1 \times 10^7$ PBMCs/ml, more preferably $2 \times 10^6$ to $5 \times 10^6$ PBMCs/ml.

**10.** Composition for the use according to any one of claims 1 to 9, wherein 0.1 to 5 ml supernatant/kg body weight, preferably 0.3 to 3 ml/kg body weight, more preferably 0.5 to 2 ml/kg body weight, more preferably 0.8 to 1.2 ml/kg body weight, is administered to a human or mammal body.

**11.** Composition for the use according to any one of claims 1 to 10, wherein the composition is administered by inhalation, topically, orally, sublingually, buccally, subcutaneously or intravenously.

**12.** Composition for the use according to any one of claims 1 to 11, wherein the mammal is a horse, a dog, a cat, or a camel.

**13.** A method for determining the suitability of a composition as defined in any one of claims 1 to 7 or 9 to be used in the treatment or prevention of a an allergy or allergic reactions caused by the administration of at least one food and/or inhalation allergen or by the systemic administration of at least one drug to a human or mammal body comprising the steps of

a) comparing skin regions of a mammal contacted with an allergen with skin regions of the same mammal contacted with the allergen and the composition as defined in any of claims 1-7 or 9,
b) identifying a difference between said regions and
c) determining whether the composition is suited to treat or prevent said disorder or disease.

**Patentansprüche**

**1.** Zusammensetzung, umfassend einen Überstand einer Zellkultur peripherer mononukleärer Blutzellen (PBMC), zur Verwendung bei der Behandlung oder Vorbeugung einer Allergie oder einer allergischen Reaktion, die durch Verabreichung mindestens eines Nahrungsmittel- und/oder Inhalationsallergens oder durch systemische Verabreichung mindestens eines Arzneimittels an einen menschlichen Körper oder einen Säugetierkörper ausgelöst wird, wobei die PBMCs vor oder während der Kultivierung ionisierender Strahlung ausgesetzt werden.

**2.** Zusammensetzung zur Verwendung gemäß Anspruch 1, wobei das mindestens eine Allergen ein biologisches oder chemisches Allergen ist.

**3.** Zusammensetzung zur Verwendung gemäß Anspruch 2, wobei das biologische Allergen ausgewählt ist aus der Gruppe bestehend aus einem tierischen Allergen, einem pflanzlichen Allergen, einem Schimmelpilz-Allergen oder einem bakteriellen Allergen.

**4.** Zusammensetzung zur Verwendung gemäß einem der Ansprüche 1 bis 3, wobei die PBMC-Zellkultur Monozyten, T-Zellen, B-Zellen und/oder NK-Zellen umfasst.

**5.** Zusammensetzung zur Verwendung gemäß einem der Ansprüche 1 bis 4, wobei die PBMCs in einem Zellkulturmedium kultiviert werden, das aus einem Zellwachstumsmedium ausgewählt ist.

**6.** Zusammensetzung zur Verwendung gemäß einem der Ansprüche 1 bis 5, wobei die PMCs einer ionisierenden Strahlung mit einer Dosis von mindestens 10 Gy, bevorzugt mindestens 20 Gy, stärker bevorzugt mindestens 40 Gy, stärker bevorzugt mindestens 50 Gy, ausgesetzt werden.

**7.** Zusammensetzung zur Verwendung gemäß einem der Ansprüche 1 bis 6, wobei die PBMCs vor der Isolierung ihres Überstands für mindestens 4 Stunden, bevorzugt für mindestens 6 Stunden, stärker bevorzugt für mindestens 12 Stunden, kultiviert werden.

**8.** Zusammensetzung zur Verwendung gemäß einem der Ansprüche 1 bis 7, wobei die Zusammensetzung vor, während und/oder nach dem Einsetzen allergischer Reaktionen und/oder dem Einwirken mindestens eines Allergens verabreicht wird.

**9.** Zusammensetzung zur Verwendung gemäß einem der Ansprüche 1 bis 8, wobei die PCBMC-Zellkultur $1\times10^5$ bis $1\times10^8$ PBMCs/ml, bevorzugt $1\times10^6$ bis $1\times10^7$ PBMCs/ml, stärker bevorzugt $2\times10^6$ bis $5\times10^6$ PBMCs/ml, umfasst.

**10.** Zusammensetzung zur Verwendung gemäß einem der Ansprüche 1 bis 9, wobei einem menschlichen Körper oder einem Säugetierkörper 0,1 bis 5 ml Überstand/kg Körpergewicht, bevorzugt 0,3 bis 3 ml/kg Körpergewicht, stärker bevorzugt 0,5 bis 2 ml/kg Körpergewicht, stärker bevorzugt 0,8 bis 1,2 ml/kg Körpergewicht, verabreicht werden.

**11.** Zusammensetzung zur Verwendung gemäß einem der Ansprüche 1 bis 10, wobei die Zusammensetzung durch Inhalation, topisch, oral, sublingual, bukkal, subkutan oder intravenös verabreicht wird.

**12.** Zusammensetzung zur Verwendung gemäß einem der Ansprüche 1 bis 11, wobei das Säugetier ein Pferd, ein Hund, eine Katze oder ein Kamel ist.

**13.** Verfahren zur Bestimmung der Eignung einer Zusammensetzung, wie in einem der Ansprüche 1 bis 7 oder 9 definiert, zur Verwendung bei der Behandlung oder Vorbeugung einer Allergie oder von allergischen Reaktionen, die durch Verabreichung mindestens eines Nahrungsmittel- und/oder Inhalationsallergens oder durch systemische Verabreichung mindestens eines Arzneimittels an einen menschlichen Körper oder einen Säugetierkörper ausgelöst wird bzw. werden, umfassend die folgenden Schritte:

a) das Vergleichen von Hautregionen eines Säugetiers, die mit einem Allergen in Kontakt gebracht wurden, mit Hautregionen desselben Säugetiers, die mit dem Allergen und der Zusammensetzung, wie in einem der Ansprüche 1-7 oder 9 definiert, in Kontakt gebracht wurden,
b) das Erkennen eines Unterschieds zwischen diesen Regionen und
c) das Bestimmen, ob die Zusammensetzung zur Behandlung oder Vorbeugung der Störung oder Krankheit geeignet ist.

## Revendications

**1.** Composition comprenant un surnageant d'une culture de cellules mononucléaires de sang périphérique (PBMC) à utiliser dans le traitement ou la prévention d'une allergie ou d'une réaction allergique provoquée par l'administration d'au moins un aliment et/ou l'inhalation d'un allergène ou par l'administration systémique d'au moins un médicament à un corps humain ou de mammifère, dans laquelle les PBMC sont soumises à un rayonnement ionisant avant ou pendant la culture.

**2.** Composition à utiliser selon la revendication 1, dans laquelle le au moins un allergène est un allergène biologique ou chimique.

**3.** Composition à utiliser selon la revendication 2, dans laquelle l'allergène biologique est choisi dans le groupe consistant en un allergène animal, un allergène végétal, un allergène de moisissure, ou un allergène bactérien.

**4.** Composition à utiliser selon l'une quelconque des revendications 1 à 3, dans laquelle la culture de cellulaire de PBMC comprend des monocytes, des lymphocytes T, des lymphocytes B et/ou des lymphocytes NK.

**5.** Composition à utiliser selon l'une quelconque des revendications 1 à 4, dans laquelle les PBMC sélectionnées sont cultivées dans un milieu de culture cellulaire, un milieu de croissance cellulaire.

**6.** Composition à utiliser selon l'une quelconque des revendications 1 à 5, dans laquelle les PBMC sont soumises à un rayonnement ionisant à une dose d'au moins 10 Gy, de préférence d'au moins 20 Gy, de manière plus préférée d'au moins 40 Gy, de manière encore plus préférée d'au moins 50 Gy.

**7.** Composition à utiliser selon l'une quelconque des revendications 1 à 6, dans laquelle les PBMC sont cultivées pendant au moins 4 h, de préférence pendant au moins 6 h, de manière plus préférée pendant au moins 12 h, avant d'isoler leur surnageant.

8. Composition à utiliser selon l'une quelconque des revendications 1 à 7, dans laquelle la composition est administrée avant, pendant et/ou après le début de réactions allergiques et/ou une exposition à au moins un allergène.

9. Composition à utiliser selon l'une quelconque des revendications 1 à 8, dans laquelle la culture cellulaire de PCBMC comprend $1{\times}10^5$ à $1{\times}10^8$ PBMC/ml, de préférence $1{\times}10^6$ à $1{\times}10^7$ PBMC/ml, de manière plus préférée $2{\times}10^6$ à $5{\times}10^6$ PBMC/ml.

10. Composition à utiliser selon l'une quelconque des revendications 1 à 9, dans laquelle de 0,1 à 5 ml de surnageant/kg de poids corporel, de préférence de 0,3 à 3 ml/kg de poids corporel, de manière plus préférée de 0,5 à 2 ml/kg de poids corporel, de manière plus préférée de 0,8 à 1,2 ml/kg de poids corporel, sont administrés à un corps humain ou de mammifère.

11. Composition à utiliser selon l'une quelconque des revendications 1 à 10, dans laquelle la composition est administrée par inhalation, par voie topique, orale, sublinguale, buccale, sous-cutanée ou intraveineuse.

12. Composition à utiliser selon l'une quelconque des revendications 1 à 11, dans laquelle le mammifère est un cheval, un chien, un chat ou un chameau.

13. Procédé pour déterminer l'aptitude d'une composition telle que définie dans l'une quelconque des revendications 1 à 7 ou 9 à être utilisée dans le traitement ou la prévention d'une allergie ou de réactions allergiques provoquées par l'administration d'au moins un aliment et/ou l'inhalation d'un allergène ou par l'administration systémique d'au moins un médicament à un corps humain ou de mammifère, comprenant les étapes consistant à

   a) comparer des régions de peau d'un mammifère mises en contact avec un allergène avec les régions de peau du même mammifère mises en contact avec l'allergène et la composition telle que définie dans l'une quelconque des revendications 1 à 7 ou 9,
   b) identifier une différence entre lesdites régions et
   c) déterminer si la composition est appropriée pour traiter ou prévenir ledit trouble ou ladite maladie.

(A)

released beta-hexosaminidase (%)

*p*<.05 Aposec vs. medium control

(B)

released beta-hexosaminidase (%)

*p*<.05 Aposec vs. medium control

Fig. 1

Fig. 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2010070105 A

- WO 2010079086 A

**Non-patent literature cited in the description**

- **HAK SUN YU et al.** Culture supernatant of adipose stem cells can ameliorate allergic airway inflammation via recruitment of CD4+CD25+Foxp3 T cells. *STEM CELL RESEARCH & THERAPY,* 23 January 2017, vol. 8 (1

- **BAIULA et al.** Mesenchymal stem cell secretome to control inflammation in allergic conjunctivitis. *Eye Sci,* 01 January 2015, 140-142
- **WAGNER T et al.** *Sci Rep.,* 2018, vol. 8 (1), 18016
- **HAIDER T et al.** *Exp Neurol.,* 2015, vol. 267, 230-42